# EUROPEAN PATENT SPECIFICATION

(11) **EP 1 292 618 B1**
(45) Date of publication and mention of the grant of the patent: **19.10.2005**
(21) Application number: 01934287.2
(22) Date of filing: 22.05.2001
(51) Int. Cl.: C07K 14/575, A61K 38/22, A61P 15/02

(54) **VIP-RELATED PEPTIDES FOR THE TREATMENT OF SEXUAL DISORDERS IN WOMEN**
VIP-VERWANDTE PEPTIDE ZUR BEHANDLUNG VON SEXUELLEN FUNKTIONSSTÖRUNGEN BEI FRAUEN
COMPOSITION PHARMACEUTIQUE COMPRENANT DES PEPTIDES ASSOCIES AU VIP DESTINEE AU TRAITEMENT DE TROUBLES SEXUELS CHEZ LES FEMMES

(30) Priority: 22.05.2000 IL 13627400; 12.10.2000 IL 13897100
(43) Date of publication of application: 19.03.2003
(73) Proprietor: Ramot at Tel Aviv University Ltd., 69975 Tel Aviv (IL); YEDA RESEARCH AND DEVELOPMENT CO. LTD., Rehovot 76100 (IL)
(72) Inventor: GOZES, Illana, 47445 Ramat Hasharon (IL); FRIDKIN, Matityahu, 76284 Rehovot (IL)
(74) Representative: Vossius & Partner
(86) International application number: PCT/IL2001/000460
(87) International publication number: WO 2001/090144

(56) References cited:
- WO-A-97/40070
- US-A- 5 998 368
- BERMAN J R ET AL: "Impotence - Basic research: Medical and non-surgical therapy: Unmoderated poster session (Tuesday, May 2) : EFFECT OF VASOACTIVE AGENTS IN MODULATING VAGINAL SMOOTH MUSCLE CONTRACTILITY-IMPLICATIONS FOR TREATMENT FEMALE SEXUAL DYSFUNCTION" JOURNAL OF UROLOGY, BALTIMORE, MD, US, vol. 163, no. 4, SUPPL, 2 May 2000 (2000-05-02), page 193 XP000982010 ISSN: 0022-5347
- LEVIN R J: "VIP, VAGINAL, CLITORAL AND PERIURETHRAL GLANS - AN UPDATE ON HUMAN FEMALE GENITAL AROUSAL" EXPERIMENTAL AND CLINICAL ENDOCRINOLOGY, LEIPZIG, DE, vol. 98, no. 2, 1991, pages 61-69, XP000891795 ISSN: 0232-7384
- OTTESEN ET AL: "Vasoactive intestinal peptide (VIP) provokes vaginal lubrication in normal women" PEPTIDES, vol. 8, 1987, pages 797-800, XP000891784

## Description

The present invention relates to the use of a lipophilic conjugate of an analogue of vasoactive intestinal peptide (VIP) wherein the methionine (Met) residue at the 17-position of VIP (Nle¹⁷-VIP) has been replaced with norleucine (Nle) for the preparation of a pharmaceutical composition for the treatment of female sexual dysfunction and/or for vaginal relaxation.

Sexual dysfunction is defined as any of a group of sexual disorders characterized by inhibition either of sexual desire or of the psychophysiological changes that usually characterize sexual response such as male erectile disorder and female sexual arousal disorder.

Sexual dysfunctions are disturbances in the sexual response cycle or pain associated with sexual arousal or intercourse. Proper sexual functioning in men and women depends on the sexual response cycle, which consists of an anticipatory mental set (sexual motive state or state of desire), effective vasocongestive arousal (erection in men; swelling and lubrication in women), orgasm and resolution.

The sexual response cycle is mediated by a delicate, balanced interplay between the sympathetic and parasympathetic nervous systems. Vasocongestion is largely mediated by parasympathetic (cholinergic) outflow; orgasm is predominantly sympathetic (adrenergic). These responses are easily inhibited by cortical influences or by impaired hormonal, neural or vascular mechanisms.

Disorders of sexual response may involve one or more of the cycle's phases. Generally, both the subjective components of desire, arousal and pleasure and the objective components of performance, vasocongestion, and orgasm are disturbed, although any may be affected independently.

Sexual dysfunction may be lifelong or acquired and may have a psychologic, physiologic or combined etiology. Thus, some diseases such as diabetes mellitus, cancer, neurologic diseases, etc. and/or some drugs such as alcohol, antihypertensives, sedatives, opioids, etc. may cause sexual dysfunction.

The most common types of sexual dysfunction in women are hypoactive sexual desire disorder, sexual arousal disorder and dyspareunia (painful coitus). In addition to the above-mentioned diseases and drugs that affect the sexual activity in both sexes, women are affected also by pelvic disorders such as endometriosis, cystitis and vaginitis, by estrogen deficiency and by oral contraceptives.

Among the methods commonly used for treatment of sexual dysfunction in women are estrogen therapy and combined estrogen/progestogen therapy. However, in view of the increased risk of endometrial cancer with estrogen therapy and the audrogenic activity of progestogens, there is a need for alternative therapies for the treatment of sexual dysfunction in women. US Patent 5,877,216 describes a method for treating sexual dysfunction in women by administration to the vagina and/or vulval area of a pharmaceutical formulation containing a selected vasodilating agent such as a prostaglandin.

Vasoactive intestinal peptide (VIP) release may induce physiological changes in sexual arousal and excitement, and may be the major neurotransmiter that participates in the innervation of the vaginal blood supply, including small blood vessels, smooth mucle and epithelial cells in the vaginal tract.

VIP has been proposed for induction of vaginal lubrication in female mammals, as described in International PCT Publication No. WO 88/03928 and corresponding Australian Patent No. 609765.

According to this disclosure, VIP is administered locally by injection to the inner wall of the vagina, or systemically by intravenous injection or by continuous infusion.

Systemic, administration of VIP to females has been found to decrease uterine smooth muscle activity and increase vaginal blood flow (Ottesen et al., Eur. J. Clin. Invest. 13, 321-324, 1983). VIP has also been found to be involved in mediating vaginal smooth muscle relaxation in female rabbits thus suggesting implications for treatment of female sexual dysfunction (Berman et al., The Journal of Urology 163, No.4, Suppl., 193, 2000). In an update on human female genital arousal, the role of VIP in vaginal blood vessel dilation and increase of blood flow of the vagina in sexual arousal is discussed (Levin, Exp. Clin. Endocrinol. 98., No. 2, 61-69, 1991). The increase of vaginal blood flow and the provocation of vaginal lubrication in normal women by VIP infusion suggests its participation in the control of physiological changes during sexual arousal: genital vasodilation and increase in vaginal lubrication (Ottesen et al., Peptides 8, 797-800, 1987). In the male, VIP induces penile erection and was suggested to induce clitorial arousal in the female (Hauser-Kronberger et al., Peptides 20, 539-543, 1999).

The present inventors have synthesized VIP derivatives, including VIP analogues and fragments, and conjugates thereof with lipophilic groups, for use in the treatment of male sexual dysfunction/impotence and for the treatment of neurodegenerative disorders (Gozes and Fridkin, J. Clin. Invest 90, 810-814, 1992; Gozes et al., Endocrinology, 134,2121-2125,1994; Gozes et al., J. Pharmacol. Exper. Therap. 273, 161-167, 1995; Gozes et al., Proc. Natl. Acad. Sci. USA 93, 427-432, 1996; Gozes et al., Proc. Natl. Acad. Sci. USA 96, 4143-4148, 1999, and US 5,147,855, US 5,998,368, EP 620008, and PCT Publication WO 97140070). These derivatives may distinguish between different VIP receptors (ibid 1995) and enhance cGMP formation. One of these derivatives, a stearoyl (St) conjugate of an analogue of VIP wherein the methionine (Met) residue at the 17-position of VIP was replaced by a norleucine (Nle) residue (herein, St-Nle¹⁷-VIP or SNV), was shown to be 1000-fold more potent than VIP in enhancing cGMP formation (Ashur-Fabian et al., Peptides 20, 629-633, 1999).

Another peptide (VIP-related) found in the urogenital tract, pituitary-adenylate cyclase activating polypeptide (PACAP), seems to be involved in stimulation of sperm motility (Gozes et al., Ann. NY Acad. Sci. USA, 865, 266-273, 1998), suggesting an added value of female sexual stimulation with VIP-PACAP-related compounds. Indeed, the short VIP fragment analogue Lys-Lys-Tyr-Leu (ibid Proc. Natl. Acad. Sci. USA, 1999) is a sequence shared by VIP and PACAP.

The limited treatment procedures for female sexual dysfunction and the side effects of these therapies strengthen the need for additional therapies.

The present invention provides the use of a lipophilic conjugate of an analogue of vasoactive intestinal peptide (VIP) wherein the methionine (Met) residue at the 17-position of VIP (Nle¹⁷-VIP) has been replaced with norleucine (Nle) for the preparation of a pharmaceutical composition for the treatment of female sexual dysfunction and/or for vaginal relaxation.

In the invention, the pharmaceutical composition is suitable for improving vaginal muscle tone and vaginal tissue health, for enhancing vaginal lubrication and enhancing sexual arousal. These activities of the VIP-related peptides will also prevent vaginal and vulvar inflammations and infections, such as bacterial, fungal and other infections of the genital tract of a female associated with insufficient vaginal lubrication activity, particularly in postmenopausal women.

In addition, the pharmaceutical composition is suitable for the treatment of vaginal relaxation, particularly, excessive vaginal relaxation.

The pharmaceutical composition is preferably for topical application in the vaginal, vulvar and/or clitorial area, in the form of a cream, gel, suspension, ointment, solution, foam or liposomal composition.

The VIP-related peptide conjugate used in the preparation of a pharmaceutical composition comprises conjugates described in US 5,147,855, US 5,998,368, EP 620008, and PCT Publication WO 97/40070.

According to the invention, the lipophilic conjugate is a conjugate of the VIP analogue coupled to a lipophilic moiety. Examples of said lipophilic group include, but are not limited to, a saturated or unsaturated carboxylic acyl having at least 5 carbon atoms selected from caproyl (Cap), lauroyl (Lau), palmitoyl, stearoyl (St), oleyl, eicosanoyl, docosanoyl, and the corresponding hydrocarbyl radicals hexyl, dodecyl, hexadecyl, octadecyl, eicosanyl, and docosanyl.

Preferred conjugates according to this embodiment of the invention include Caproyl-norleucine¹⁷-VIP (Cap-Nle¹⁷-VIP), and, more preferably, Stearoyl-norleucine¹⁷-VIP (St-Nle¹⁷-VIP, also designated herein SNV).
- **Fig. 1**: shows that 0.3 µM VIP neutralizes vaginal contraction.
- **Fig. 2**: shows that 0.3 µM SNV attenuated the rate of vaginal contraction stimulated by 0.5 µM phenylephrine. In addition, SNV administration also reduced the contraction force (negative inotropic effect).
- **Fig. 3**: shows that 1.5 µM SNV blocked vaginal contraction stimulated by 0.5 µM phenylephrine.

The present invention provides the preparation of pharmaceutical compositions, in particular, topical formulations of a small VIP-related peptide as disclosed above in form of conjugates with lipophilic groups, for non-invasive treatment of female sexual dysfunction and/or for vaginal relaxation.

The aspect of the vaginal relaxation is of interest in cases of pregnant women prone to abortion. This treatment is intended to prevent abortion in such women.

The compositions of the invention are intended for topical vaginal, vulvar or clitoral administration. The "***pharmaceutically acceptable carrier***" of the pharmaceutical composition is a material suitable for vaginal, vulvar or clitoral delivery that is non-toxic and does not affect negatively the active ingredient or any other component of the formulation. The carrier is preferably selected from amongst those which enhance the tissue penetration of the active ingredient and include, without being limited to, carriers such as glycerine, lubricants, olive oil, nitroglycerine, glyceryl monocaprylate, propylene glycol didecanoate, propylene glycol dicaprylate, glyceryl tricaprylate, sorbitan monocaprylate, and mixtures thereof.

Other compositions suitable for local administration are as previously described (Olcada et al., J. Pharmaceut. Sci. 71, 1367-1371, 1982) and may include jelly, starch, or protease inhibitors, or Sefsol™ /isopropanol (Gozes et al., Endocrinology, 134,2121-2125,1994). Pharmaceutical creams, viscous liquid or semi-solid emulsion containing oil phase and water-based phase can be prepared as described in Remington's Pharmaceutical Sciences, 18^{th} Ed., Easton, PA: Mack Publishing Company, 1990. Other suitable form for drug delivery is as liposomes (microscopic vesicles having a lipid wall comprising a lipid bilayer, e.g. lipofactin, GIBCO, BRL, Grand Island, NY).

An effective amount of the pharmaceutically active ingredient used in the invention is an amount that is sufficient to provide the desired degree of treatment.

The invention is preferably applied to human females, although it can also be applied to other species, such as bovine, canine, equine, ovine and porcine.

The composition is preferably locally administered to a portion of the wall of the female genital tract, most preferably to a portion of the inner wall of the vagina. Local administration can be accomplished by diffusion from a solution dispersed in a suitable support, such as a porous tampon, a suppository made with a composition comprising oleaginous base materials, or a suitable composition such as emulsion, cream, jelly, or tablet or using a applicator such as applicators used to self-administer contraceptive foam.

The invention will now be illustrated, by the following non-limitative Examples.

### EXAMPLE 1

### Synthesis of the VIP-related peptides

The VIP-related peptide as part of a conjugate used in the compositions prepared in the invention is prepared by standard peptide synthesis procedures well known in the art. The conjugates with a lipophilic group are prepared substantially as described in US 5,147,855, US 5,998,368, EP 620008, and PCT Publication WO 97/40070.

### EXAMPLE 2

### Cream and ointment formulations

Cream and ointment formulations comprising the VIP-related peptide stearoyl-Nle¹⁷VIP (SNV) are prepared substantially as described in US Patent No. 5,877,216, US Patent No. 5,998,368, and PCT WO 97/40070.

The concentration of the VIP-related peptide in the physiologically acceptable solution is between 0.01 micrograms and about 100 micrograms per ml.

### EXAMPLE 3

Candidate sexual dysfunction patients are assembled from a group of female individuals assessed and prescreened. Following topical administration of a composition of the invention to the vagina, vulvar area or clitoris, changes in uterine or vaginal epithelial blood flow are measured by known methods. Increase in vaginal epithelial blood flow is measured by photoplethysmography (Levin et al., 1980, Clinics in Obstet. Gynaecol. 7: 213-252), heated oxygen electrode (Wagner et al., 1978, "Vaginal Fluid" in The Human Vagina, Evans et al. (eds), Amsterdam, Elsevier, North Holland Biomedical Press, pp. 121-137), and direct clearance of radioactive Xenon (Wagner et al., 1980, Obstet. Gynaecol. 56: 621-624). Changes in vulvar blood flow are measured using Doppler velocimetry (US Patent 5,877,216, Australian Patent 609765), and increased lubrication is measured using pre-weighed circular filter papers arranged in a layer and held inside a plastic suction capsule and in direct contact with the vaginal wall, additional fluid is measured by weight at determined times after application (Australian Patent 609765).

### EXAMPLE 4

Smooth muscle tone regulation in rabbit cavernosal and spongiosal tissue follows the method described by Spawasser et al., J. Urology 152,2159-2163, 1994. Briefly, rabbit is used as a model (Taub et al., Urology, 42,698-704, 1993), and longtitudinal strips (cavernous and urethral) are obtained, and mounted between two metal hooks in an organ bath chamber containing 10 ml of Krebs solution. Tension is measured by an adjustable connection to a Grass Instrument force-displacement transducer. Strips are preconditioned with phenylephrine at a concentration of 3×10⁶ mol./l (40% maximal contraction). Control uses papaverine (Chen, K.-K., et al., J. Urology 147,1124-1128, 1992; Spawasser et al. 1994, ibid), alprostadil and VIP1-28. All experiments comprise dose-response curves. Similar experiments are conducted with female rabbit vaginal and clitorial smooth muscle (Ottesen et al., 1983, ibid), or utilizing a rabbit clitorial smooth muscle cell culture to study receptor binding and direct effects (H. Sadehi-Nejad et al., International J. Imp. Res. 10, 165-169, 1998). Blood flow is measured in a rabbit animal model as described before (park et al., International J. Imp. Res. 9, 27-37, 1997).

### EXAMPLE 5

Cyclic GMP has been associated with smooth muscle relaxation and hence directly implicated in sexual arousal and increased blood flow to the sex organs. SNV is 1000-fold more potent than VIP in inducing cGMP formation (Ashur-Fabian 1999, ibid). Other VIP-related peptides (Gozes et al., 1999 ibid) are tested in a similar assay system.

### EXAMPLE 6

The 28 amino acid, vasoactive intestinal peptide (VIP) may induce the physiological changes in sexual arousal and excitement, and may be the major neurotransmitter that participates in the innervation of the vaginal blood supply, including small blood vessels, smooth muscle and epithelial cells in the vaginal tract (US Patent 5,877,216; Australian patent 609765). Systemic administration of VIP to females has been found to decrease uterine smooth muscle activity and increase vaginal blood flow (Ottesen et al., Eur. J. Clin. Invest. 13, 321-324,1983). In males, VIP induces penile erection and is thus suggested to induce clitorial arousal in the female (Hauser-Kronbrger et al., Peptides 20, 539-543, 1999). VIP derivatives and conjugates were designed to include a lipophilic moiety or a shortened VIP chain (Gozes and Fridkin, J. Clin. Invest. 90, 810-814, 1992; Gozes et al., Endocrinology, 134,2121-2125,1994; Gozes et al., J. Pharmacol. Exper. Therap. 273, 161-167, 1995; Gozes et al., Proc. Natl. Acad: Sci. USA 93, 427-432, 1996; Gozes et al., Proc. Natl. Acad. Sci. USA 96, 4143-4148, 1999). These derivatives may distinguish different VIP receptors (Gozes et al., 1995 ibid.) and enhance cGMP formation, 1000-fold more potently than VIP (Ashur-Fabian et al., Peptides 20, 629-633, 1999). Interestingly, the proposed mechanism of action of Sildenafil Citrate is through inhibition of cGMP breakdown (Moreland et al., Trends Endocrinol Metab. 10, 97-104, 1999). Given the proposed .mechanism of action (Gozes and Fridkin, 1992, ibid.; Gozes et al., 1994 ibid.; Gozes et al., 1995, ibid.; Gozes et al., 1996, ibid.; Gozes et al., 1999, ibid.; Ashur-Fabian et al., 1995, ibid) and the increased bioavailability (Gozes and Fridkin, 1992, ibid.; Gozes et al., 1994, ibid.), lipophilic VIP derivatives are now suggested as potential topical treatment for female sexual dysfunction. The first compound chosen for further investigations was Stearoyl-Nle¹⁷VIP = SNV, comprising the VIP molecule with two modifications, namely an N-terminal attachment of stearic acid residue and an exchange of the methionine residue in position 17 with norleucine. SNV exhibits increased stability and bioavailability (Gozes et al., 1994, ibid.). SNV was now tested for its ability to induce vaginal relaxation.

### Methods:

The vagina of 12-week-old New Zealand female rabbit was removed, exiced to 1 cm length and immersed in 10 ml of capacity organ bath connected to an external reservoir of Krebs-Henseleit in constant temperature of 37°C. The solution was oxygenated (95%O₂/25%CO₂). The effect of VIP and SNV on vaginal contraction was measured by attaching the vaginal muscle to a force transducer.

### Results:

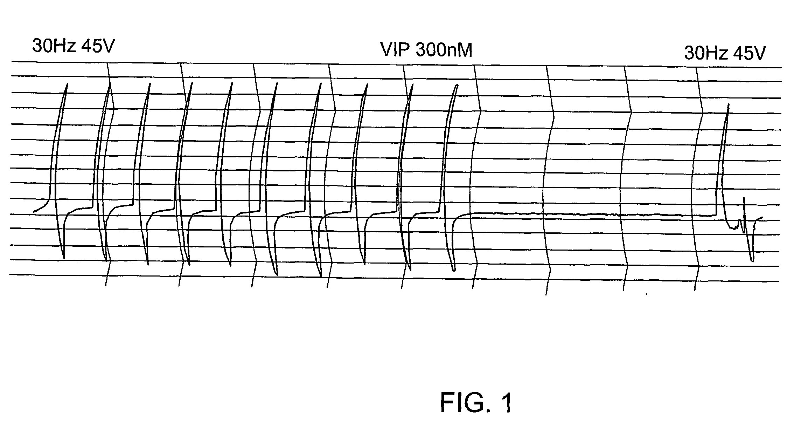

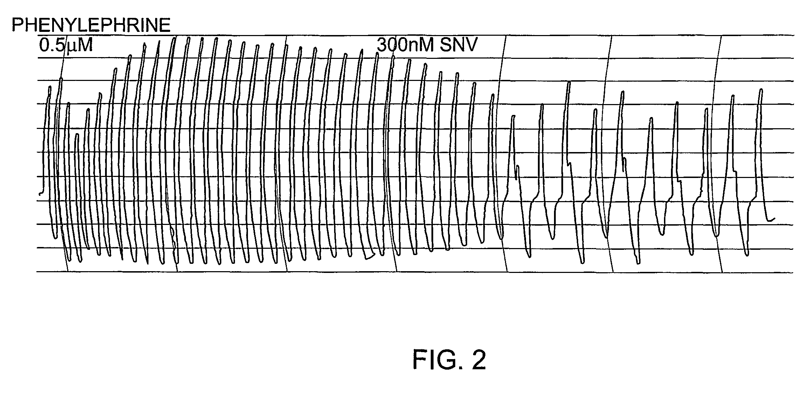

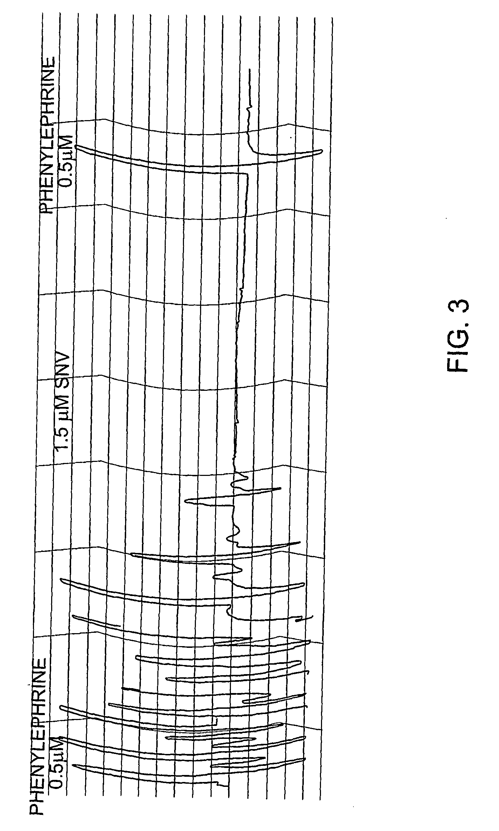

Preliminary results showed that 0.3 µM of VIP neutralized vaginal contraction (Fig. 1). At the same concentration, SNV attenuated the rate of contraction, stimulated by 0.5 µM phenylephrine (Fig. 2). In addition, SNV administration also reduced the contraction force (negative inotropic effect). High concentrations of SNV (1.5 µM) neutralized vaginal contraction stimulated by 0.5 µM phenylephrine (Fig. 3).

From these experiments we can conclude that SNV may protect against excessive vaginal dilatation and that SNV holds promise for the treatment of female sexual dysfunction.

### SEQUENCE LISTING

<110> Ramot University Authority
   Yeda Research and Development Company Ltd.
<120> PHARMACEUTICAL COMPOSITIONS COMPRISING VIP-RELATED
   PEPTIDES FOR THE TREATMENT OF SEXUAL DISORDERS
<130> 1330943- YEDA+RAMOT
<140>
   <141>
<150> IL 136274
   <151> 2000-05-22
<150> IL 138971
   <151> 2000-10-12
<160> 21
<170> PatentIn Ver. 2.1
<210> 1
   <211> 25
   <212> PRT
   <213> Homo sapiens
<400> 1
   His Ser Asp Ala Phe Thr Asp Asn Tyr Thr Arg Leu Arg Lys Gln Ala
     1               5                  10                  15
   Lys Lys Tyr Leu Asn Ser Ile Leu Asn
                20                  25
<210> 2
   <211> 25
   <212> PRT
   <213> Homo sapiens
<400> 2
   His Ser Asp Ala Phe Thr Asp Asn Tyr Thr Arg Leu Arg Lys Gln Ala
     1               5                  10                  15
   Lys Lys Tyr Leu Asn Ser Ile Leu Asn
                20                  25
<210> 3
   <211> 11
   <212> PRT
   <213> Homo sapiens
<400> 3
   Ala Val Lys Lys Tyr Leu Asn Ser Ile Leu Asn
     1               5                  10
<210> 4
   <211> 4
   <212> PRT
   <213> Homo sapiens
<400> 4
   Lys Lys Tyr Leu
     1
<210> 5
   <211> 3
   <212> PRT
   <213> Homo sapiens
<400> 5
   Lys Lys Tyr
   1
<210> 6
   <211> 5
   <212> PRT
   <213> Homo sapiens
<400> 6
   Val Lys Lys Tyr Leu
   1 5
<210> 7
   <211> 6
   <212> PRT
   <213> Homo sapiens
<400> 7
   Ala Val Lys Lys Tyr Leu
   1 5
<210> 8
   <211> 5
   <212> PRT
   <213> Homo sapiens
<400> 8
   Asn Ser Ile Leu Asn
   1 5
<210> 9
   <211> 4
   <212> PRT
   <213> Homo sapiens
<400> 9
   Lys Lys Tyr Val
   1
<210> 10
   <211> 4
   <212> PRT
   <213> Homo sapiens
<400> 10
   Ser Ile Leu Asn
   1
<210> 11
   <211> 5
   <212> PRT
   <213> Homo sapiens
<400> 11
   Asn Ser Tyr Leu Asn
   1 5
<210> 12
   <211> 5
   <212> PRT
   <213> Homo sapiens
<400> 12
   Asn Ser Ile Tyr Asn
   1 5
<210> 13
   <211> 3
   <212> PRT
   <213> Homo sapiens
<400> 13
   Ala Val Lys
   1
<210> 14
   <211> 3
   <212> PRT
   <213> Homo sapiens
<400> 14
   Lys Tyr Leu
   1
<210> 15
   <211> 3
   <212> PRT
   <213> Homo sapiens
<400> 15
   Lys Lys Tyr
   1
<210> 16
   <211> 5
   <212> PRT
   <213> Homo sapiens
<400> 16
   Ala Val Lys Lys Tyr
   1 5
<210> 17
   <211> 5
   <212> PRT
   <213> Homo sapiens
<400> 17
   Val Lys Lys Tyr Leu
   1 5
<210> 18
   <211> 5
   <212> PRT
   <213> Homo sapiens
<400> 18
   Leu Asn Ser Ile Asn
   1 5
<210> 19
   <211> 6
   <212> PRT
   <213> Homo sapiens
<400> 19
   Tyr Leu Asn Ser Ile Asn
   1 5
<210> 20
   <211> 4
   <212> PRT
   <213> Homo sapiens
<400> 20
   Lys Lys Tyr Leu
   1
<210> 21
   <211> 5
   <212> PRT
   <213> Homo sapiens
<400> 21

Note : Only the use of the peptide of Claim 1 is part of this invention.

## Claims

1. Use of a lipophilic conjugate of an analogue of vasoactive intestinal peptide (VIP) wherein the methionine (Met) residue at the 17-position of VIP (Nle¹⁷-VIP) has been replaced with norleucine (Nle) for the preparation of a pharmaceutical composition for the treatment of female sexual dysfunction and/or for vaginal relaxation.

2. Use according to claim 1, wherein said lipophilic group of said conjugate is a saturated or unsaturated carboxylic acyl having at least 5 carbon atoms selected from caproyl (Cap), lauroyl (Lau), palmitoyl, stearoyl (St), oleyl, eicosanoyl, docosanoyl, and the corresponding hydrocarbyl radicals hexyl, dodecyl, hexadecyl, octadecyl, eicosanyl, and docosanyl.

3. Use according to claim 2, wherein said lipophilic group is selected from stearoyl, caproyl and lauroyl.

4. Use according to claim 3, of a stearoyl conjugate of Nle¹⁷-VIP-(SNV).

5. Use according to any one of claims 1 to 4, for improving vaginal muscle tone and vaginal tissue health, for enhancing vaginal lubrication and enhancing sexual arousal.

6. Use according to any one of claims 1 to 5 wherein the pharmaceutical composition is for topical application in the vaginal, vulvar and/or clitorial area.

7. Use according to claim 6, wherein the pharmaceutical composition is in the form of a cream, gel, suspension, ointment, solution, foam or liposomal composition.

## Patentansprüche

1. Verwendung eines lipophilen Konjugats eines Analogs des vasoaktiven intestinalen Peptids (VIP), in dem der Methionin (Met)-Rest an der Position 17 von VIP (Nle¹⁷-VIP) mit Norleucin (Nle) ersetzt worden ist, für die Herstellung eines Arzneimittels zur Behandlung von weiblicher sexueller Funktionsstörung und/oder zur Entspannung der Scheide.

2. Verwendung nach Anspruch 1, wobei die lipophile Gruppe des Konjugats ein gesättigtes oder ungesättigtes Carboxyacyl mit mindestens 5 Kohlenstoffatomen ist, ausgewählt aus Caproyl (Cap), Lauroyl (Lau), Palmitoyl, Stearoyl (St), Oleyl, Eicosanoyl, Docosanoyl und den entsprechenden Kohlenwasserstoffresten Hexyl, Dodecyl, Hexadecyl, Octadecyl, Eicosanyl und Docosanyl.

3. Verwendung nach Anspruch 2, wobei die lipophile Gruppe ausgewählt ist aus Stearoyl, Caproyl und Lauroyl.

4. Verwendung eines Stearoyl-Konjugats von Nle¹⁷-VIP (SNV) nach Anspruch 3.

5. Verwendung nach einem der Ansprüche 1 bis 4 zum Verbessern des vaginalen Muskeltonus und der Gesundheit des Vaginalgewebes, zum Verstärken der vaginalen Feuchtigkeit und zum Verstärken der sexuellen Erregung.

6. Verwendung nach einem der Ansprüche 1 bis 5, wobei das Arzneimittel zur topischen Anwendung in der Vagina, Vulva und/oder im Bereich der Klitoris ist.

7. Verwendung nach Anspruch 6, wobei das Arzneimittel in Form einer Creme, eines Gels, einer Suspension, einer Salbe, einer Lösung, eines Schaums oder einer liposomalen Zusammensetzung ist.

## Revendications

1. Utilisation d'un conjugué lipophile d'un analogue du peptide intestinal vasoactif (VIP) dans lequel le résidu méthionine (Met) en position 17 du VIP (Nle¹⁷-VIP) est remplacé par la norleucine (Nle) pour la préparation d'une composition pharmaceutique pour traiter un dysfonctionnement sexuel féminin et/ou pour la relaxation vaginale.

2. Utilisation selon la revendication 1, dans laquelle le groupe lipophile dudit conjugué est un acyl carboxylique saturé ou insaturé ayant au moins 5 atomes de carbone sélectionné parmi caproyl (Cap), lauroyl (Lau), palmitoyl, stéaroyl (St), oléyl, eicosanoyl, docosanoyl, et les radicaux hydrocarbonés correspondants hexyl, dodécyl, hexadécyl, octadécyl, eicosanyl et docosanyl.

3. Utilisation selon la revendication 2, dans laquelle le groupe lipophile est sélectionné parmi stéaroyl, caproyl et lauroyl.

4. Utilisation selon la revendication 3, d'un conjugué stéaroyl du Nle¹⁷-VIP (SNV).

5. Utilisation selon l'une des revendications 1 à 4, pour améliorer la tonicité du muscle vaginal et la santé tissulaire du vagin, pour améliorer la lubrification vaginale et améliorer l'excitation sexuelle.

6. Utilisation selon l'une des revendications 1 à 5, dans laquelle la composition pharmaceutique est destinée à une application topique dans la partie vaginale, vulvaire et/ou du clitoris.

7. Utilisation selon la revendication 6, dans laquelle la composition pharmaceutique est sous forme de crème, gel, suspension, onguent, solution, savon ou composition liposomale.
